# EUROPEAN PATENT APPLICATION

(11) **EP 1 043 331 A1**
(43) Date of publication of application: **11.10.2000**
(21) Application number: 98961589.3
(22) Date of filing: 25.12.1998
(51) Int. Cl.: C07J 41/00, A61K 31/565

(54) **BONE-RESORPTIVE CYTOKINE INHIBITOR CONTAINING DIENOGEST AS THE ACTIVE INGREDIENT**

(30) Priority: 26.12.1997 JP 36112497
(71) Applicant: MOCHIDA PHARMACEUTICAL CO., LTD., Shinjuku-ku Tokyo 160-8515 (JP)
(72) Inventor: SHIBUTANI, Yasunori, Mochida Phar. Co., Ltd., Tokyo 160-8515 (JP); FUTAMURA, Yoshihiro, Mochida Phar. Co., Ltd., Tokyo 160-8515 (JP); KATSUKI, Yukio, Mochida Phar. Co., Ltd., Tokyo 160-8515 (JP)
(74) Representative: Grünecker, Kinkeldey, Stockmair & Schwanhäusser Anwaltssozietät
(86) International application number: JP9805945
(87) International publication number: WO9933857

(57) **Abstract**

Providing dienogest and a solvate thereof, having a novel action to suppress cytokine production and a method for utilizing the same.

An agent to suppress the production and/or secretion of IL-1, IL-6 and TNF-α, wherein the agent characteristicaly contains dienogest or a solvate thereof as the effective ingredient, and a prophylactic and/or therapeutic agent of chronic rheumatoid arthritis, sosteoarthritis, and hypercalcemia following malignant tumor.

## Description

### Technical Field

The present invention relates to an agent for suppressing the production and/or secretion of interleukin 1 (referred to as "IL-1" hereinbelow), interleukin 6 (referred to as "IL-6" hereinbelow), and tumor necrosis factor α (referred to as "TNF-α" hereinbelow), wherein the agent contains dienogest as the effective ingredient.

### Background Art

Dienogest is the International Nonproprietary Name (INN) of a known compound with the following structure (17 α-cyanomethyl-17β-hydroxy-estra-4,9(10)-dien-3-one) having a structure represented by the following formula (I).

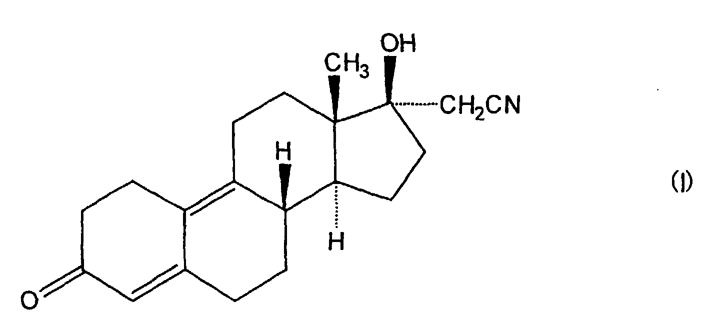

The properties of the compound and a method for synthesizing the compound are generally described by Shubert, et al., Natural Products Chemistry 1984, Elsevier Science Publishers eds., 1985, pp.143-158.

Dienogest has been known to have progestational activity. In Germany, a combined drug of dienogest with ethinylestradiol has been introduced as an oral contraceptive agent into market. Additionally, the clinical development of dienogest as a therapeutic agent of endometriosis is ongoing (Kohler et al., Archives of Gynecology and Obstetrics, Vol.254, pp.594-595, 1993), and experimental reports have been issued to demonstrate the carcinostatic action of dienogest on uterine cancer and breast cancer (Katsuki et at., Japanese Patent Laid-open No. Hei 7-188026; Katsuki, et al., Cancer, Vol.79, pp.169-176, 1997). Still furthermore, dienogest has a protective action against bone reduction due to ovariectomy in rats (Elger et al., Experimental Clinical Endocrinology, Suppl. Vol.101, No.1, pp.71, 1993). It has been reported that if dienogest is used in combination with buserelin which is a GnRH agonist, dienogest can work to restore the reduction of bone density, due to buserelin, to the general level (Sasagawa et al., Japanese Journal of Pharmacology, Vol.67 Suppl., p.162, 1995). Up to now, however, the action mechanism has not yet been elucidated, or no report has been published yet, telling that dienogest affects the suppression of the production and secretion of cytokine.

Cytokine means proteinous active substances mainly produced in leukocyte, excluding antibodies, and the action is multiple, primarily including functions relating to the control and regulation of immune system and transmission, functions relating to the differentiation and induction of hematopoietic stem cell to the proliferation thereof and functions responsible for inflammatory response. The common characteristic properties of cytokine are as follows; cytokine exerts specific functions at a trace amount, and activates the functions of various types of cells including the action on the cells generating cytokine, and exhibits important functions on biological prevention and immune response.

TNF-α was firstly observed in sera of mice given with endotoxin, and it was named as tumor necrosis factor because it caused hemorrhagic necrosis of a certain type of tumor. Main action includes the induction of cytokine primarily including IL-1, IL-6 and IL-8, the activation of phospholipase, the production of arachidonic acid metabolites and platelet activating factor, the differentiation and induction of hematopoietic stem cell, the acceleration of hormone secretion, and pyrexia.

It has been also noted in recent years that TNF-α is involved in peripheral insulin resistance in muscles and the like.

IL-1 divided as two types of IL-1α and IL-1β was firstly found as a lymphocyte activating factor produced by macrophage. After in vivo administration thereof, blood pressure reduction is observed, together with pyrexia, sleepiness neutropilia, the increase of hormonal secretion, and the production and induction of protein at an acute phase.

IL-6 was firstly found as a humoral factor (B-cell activating factor) produced by macrophage or by T lymphocyte, which is required for the proliferation and differentiation of B lymphocyte on antigen stimulation. IL-6 also has multiple biological activities and functions. It triggers the induction of antibody production, T lymphocyte activation, the induction of acute phase response, the induction of megakaryocyte maturation, and the activation of osteoclast and the like. It is said on the basis of what has been described above that the acceleration of the production of IL-6 has close relation with chronic rheumatoid arthritis (referred to as "RA" hereinbelow), atrial myxoma, Castleman's disease, hyper γ-globulinemia in AIDS and the like and Kaposi's sarcoma in AIDS, mesangium proliferation nephritis, psoriasis, sepsis and osteoporosis and the like.

RA has been treated conventionally by using anti-inflammatory agents, modifying anti-rheumatoid agents with modification potency, immune suppressing agents, and immunomodulators and the like, but the therapeutic treatment with them is not satisfactory. Additionally because no etiology has been elucidated yet, no definitive therapeutic method thereof has been found. Research works insofar possibly indicate that IL-6 as one type of cytokine has a fairly close relation with RA. Actually, IL-6 is detected in the synovial fluid (joint fluid) of RA patients. It is said that IL-6 stimulates antibody formation and is responsible for the occurrence and increase of rheumatoid factor, and hyper γ-globulinemia, as well as for the activation and increase of T lymphocyte in joint lesions, and is additionally responsible for bone fracture. Similarly, TNF-α and IL-1 are also detected in the synovial fluid of RA patients and are responsible for the progress of pathological conditions.

At its early stage, RA simply causes inflammation of synovial membrane, with symptoms such as morning stiffness of joints of hands and legs, systemic malaise, easy fatigue, body weight decrease, the enlargement of lymphatic nodule, and hypodermic nodule. Further progress involves the fracture of cartilage and bone, while joints are deformed and dislocated, eventually leading to sequela such as stiffness and rigidity, which severely affects daily life and motion.

It has been elucidated in recent years that a great number of various types of cytokine are involved in the physiological remodeling of bone or the occurrence of pathological conditions due to the damage. The cytokine responsible for bone metabolism includes cytokine with bone resorption promoting action, such as IL-1, IL-6 , TNF, macrophage stimulating factor (M-CSF), leukemia inhibitory factor (LIF), IL-11 and the like; cytokine with bone resorption suppressing action, such as IL-4, and IFN-γ; cytokine with osteogenesis promoting action such as bone morphological factor (BMP) and transforming growth factor (TGF)-β and the like. The IL-1 and IL-6 are known as osteoclast production promoting factor; and TNF-α is known as a regulating factor of osteoclast. These bone resorption accelerating factors promote bone resorption, through the differentiation and induction of osteoclast, the activation of mature osteoclast, and the activation of osteoclast through osteoblast. On stimulation with IL-1 and the like, osteoblast induces the production of osteoclast differentiation factor and PG, to consequently induce the differentiation and activation of osteoclast. Furthermore because estrogen suppresses the level of bone-resorptive cytokine such as IL-1, IL-6 and TNF-α, such agent to suppress the production and/or secretion of those bone resorption acceleration factors is promising as the agents to prevent or therapeutically treat postmenopausal osteoporosis which results from the excess acceleration of bone resorption.

Osteoporosis is divided as postmenopausal osteoporosis, senile osteoporosis and secondary osteoporosis. Because postmenopausal osteoporosis is caused by the deletion of estrogen after menopause, resulting in the excess acceleration of bone resorption, bone resorption suppressive agents such as calcitonin or estrogen are used as the therapeutic agents therefor. However, calcitonin is problematic in that the dosage is limited to intra-muscular administration and that calcitonin tolerance readily occurs; and estrogen has a drawback of the increase of the frequencies of breast cancer and uterus endometrial cancer. Because the decrease of osteogenesis due to aging is significant as the etiology of senile osteoporosis, alternatively, those with an action to activate deteriorated osteoblast functions (osteogenesis), namely so-called osteogenesis promoting agents, are effective as the therapeutic agents thereof. More specifically, the agents include vitamin D as well as sodium fluoride, vitamin K, and human PTH (parathyroid hormone) formulations.

It has been reported that the increase of cyclic AMP (referred to as "cAMP" hereinbelow) content and the activation of protein kinase A (referred to as "PKA" hereinbelow) have some relation with PTH with the known action to stimulate the proliferation or to promote the differentiation of osteoblast (Yang et al., Journal of Biological Chemistry, Vol.271, pp.29839-846, 1996; Ahlstrom et al., Journal of Bone Mineral Research, Vol.12, pp.172-178, 1997). More specifically, it is believed that the elevation of cAMP content level and the activation of PKA activate the function of osteoblast, which serves to promote osteogenesis. Because no sufficiently effective therapeutic agent is currently present in terms of the increase of bone in patients with osteoporosis, attention is now focused toward the agent of promoting osteogenesis as a therapeutic agent of senile osteoporosis.

Irrespective of age and sex, secondary osteoporosis occurs following specific diseases including endocrine diseases and inflammatory diseases, and is cured mildly after the etiology is eliminated. Specifically, the etiology of secondary osteoporosis lies in endocrine and metabolic diseases such as hyperparathyroidism and diabetes mellitus; inflammatory diseases such as RA; hematological diseases such as multiple myeloma; gastro-intestinal diseases such as cirrhosis; and congenital diseases such as osteogenesis impairment. The pathological conditions vary more or less, depending on the primary disease, but generally, the conditions include normal osteogenesis with accelerated bone resorption, normal bone resorption with deteriorated osteogenesis, and the mixture of the two. Therefore, an agent only with a bone resorption suppressing action is not satisfactory as a preventive and therapeutic agent.

It is believed that osteoarthritis is a pathological condition where the degeneration and damage of joint cartilage and the proliferation mechanism of bone cartilage therearound simultaneously progress, accompanying secondary synovial inflammation, with the main symptoms of knee pain, motion regulation, hydrarthrosis, and deformation and the like. Knee osteoarthritis in particular is a therapeutically significant disease. As the treatment, preservative treatment is of a first choice for mild to moderate patients; and surgery treatment is done for moderate to severe patients.

When GnRH agonists are used so as to decrease estrogen for treating endometriosis and uterine leiomyoma, a side effect of the reduction of bone mineral density occurs. For the purpose of reducing the side effect, therefore, the concurrent use of medroxyprogesterone acetate (referred to as "MPA" hereinbelow) is known. However, the effect is not satisfactory even at a higher dose, and thrombosis and the like occur disadvantageously as the side effects. Additionally, the combined use of MPA and estrogen suppresses the reduction of bone mineral density, but it is reported that the single use of MPA reduces spinal bone mineral density (Gallagher, et al., American Journal of Medicine, Vol.90, pp.171-178, 1991). Furthermore, the relation of MPA with bone-resorptive cytokine is not apparently elucidated.

Furthermore, danazol for use in therapeutically treating endometriosis and uterine leiomyoma as well as tamoxifen for use in treating breast cancer has a drawback of such side effect of bone reduction.

In addition, as to the relevance of TNF-α to the peripheral insulin resistance, it is known that TNF-α produced in fat cells induces the insulin resistance in the adjacent muscular cells or that TNF-α produced by the muscle itself is also involved in the insulin resistance (Sato, Latest Medicine, Vol. 52, No. 6, pp. 1131 - 1136, 1997). Therefore, agents for suppressing the production and/or secretion of TNF-α are started to be used to ameliorate the insulin resistance, whereupon diabetes mellitus is treated or impaired glucose tolerance is treated for the prevention or retardation of the onset of non-insulin-dependent diabetes mellitus (NIDDM). The agent is now typified by thiazolidine derivatives, which still are not satisfactory in such aspects as the side effects and the like.

### Disclosure of the Invention

In such circumstances, it is demanded to develop an agent with less side effects, which can be given for a long time and which is highly effective for diseases with the etiology of the abnormality in production and secretion of bone-resorptive cytokine, particularly for RA, osteoarthritis, hypercalcemia following malignant tumor, osteoporosis and diabetes mellitus. The present invention resolves at least one of above symptoms.

The present inventors have earnestly investigated to solve the problems, and found that dienogest unexpectedly has an action to suppress the production and secretion of bone-resorptive cytokine. The present inventors also have found that dienogest has an action to promote osteogenesis in osteoblast. Hence, the present invention has been achieved.

A first aspect of the present invention is an agent to suppress the production and/or secretion of at least one of IL-1, IL-6 and TNF-α, wherein the agent contains dienogest and/or a solvate thereof as the effective ingredient. Preferably, the agent is an agent to suppress the production and/or secretion of IL-6, while the agent still retains the aforementioned characteristics.

A second aspect of the present invention is an agent to suppress the production and/or secretion of bone-resorptive cytokine, wherein the agent contains dienogest or a solvate thereof as the effective ingredient. Preferably, the bone-resorptive cytokine is at least one of IL-1, IL-6 and TNF-α. More preferably, the cytokine is IL-6.

A third aspect of the present invention is an agent to suppress the production and/or secretion of bone-resorptive cytokine in osteoblast, wherein the agent contains dienogest or a solvent thereof as effective ingredient. Preferable bone-resorptive cytokine in osteoblast is at least one of IL-1, IL-6 and TNF-α, and more preferable one is IL-6.

A fourth aspect of the present invention is a prophylactic and/or therapeutic agent of diseases due to the abnormality in production and/or secretion of at least one of IL-1, IL-6 and TNF-α, wherein the agent contains dienogest or a solvate thereof as the effective ingredient. More specifically, the agent is a prophylactic and/or therapeutic agent of the diseases due to the abnormality in production and/or secretion of at least one of IL-1, IL-6 and TNF-α, which diseases are among at least one of RA, osteoarthritis, knee osteoarthritis, hypercalcemia following malignant tumor, senile osteoporosis, secondary osteoporosis and diabetes mellitus, interalia NIDDM.

A fifth aspect of the present invention is a prophylactic and/or therapeutic agent of at least one of diseases of RA, osteoarthritis, knee osteoarthritis, hypercalcemia following malignant tumor, senile osteoporosis and secondary osteoporosis, wherein the agent contains dienogest or a solvate thereof as the effective ingredient. Preferably, the agent, having the aforementioned characteristic properties, is a prophylactic and/or therapeutic agent of RA, or a prophylactic and/or therapeutic agent of senile osteoporosis.

A sixth aspect of the present invention is an agent to suppress bone resorption in osteoclast, or an agent to induce apoptosis in osteoclast, wherein the agent contains dienogest or a solvate thereof as the effective ingredient.

A seventh aspect of the present invention is an agent to promote osteogenesis, wherein the agent contains dienogest or a solvate thereof as the effective ingredient.

An eighth aspect of the present invention is an agent to increase cAMP content in osteoblast and elevate PKA activity, wherein the agent contains dienogest or a solvate thereof as the effective ingredient.

A ninth aspect of the present invention is an agent to reduce side effects, wherein the agent is used in combination with danazol.

A tenth aspect of the present invention is an agent to suppress the production and/or secretion of bone-resorptive cytokine, which agent characteristically contains dienogest or a solvate thereof as the effective ingredient and is useful as a reagent.

The fourth, fifth and ninth aspects of the present invention include a prophylactic and/or therapeutic method of each disease or a method for reducing such side effects, using a pharmaceutical composition which contains dienogest or a solvate thereof as the effective ingredient. A use of dienogest or a solvate thereof in the production of a prophylactic and/or therapeutic agent of each disease or an agent for reducing such side effects is also included.

### Brief Description of Drawings

Fig. 1 presents Graphs depicting cAMP content in the Experimental Example 4.
Fig. 2 presents Graphs depicting PKA activity in the Experimental Example 5.

### Best Mode for Carrying out the Invention

The present invention will be described in detail below.

As already shown above, the dienogest as the effective ingredient of the agent to suppress the production and secretion of bone-resorptive cytokine in accordance with the present invention is a compound with the structure represented by the formula (I), and can form a solvate with pharmaceutically acceptable various solvents such as water, ethanol, glycerol and acetic acid.

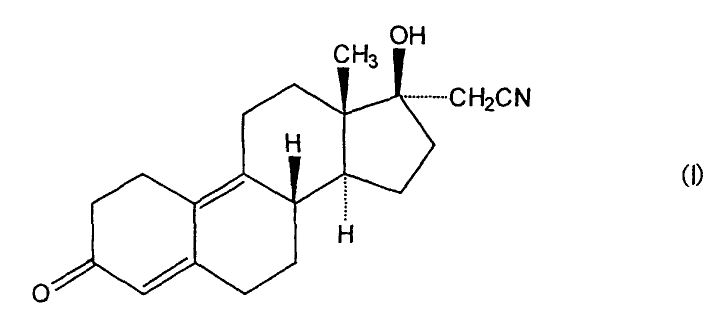

Dienogest has an action to suppress the production and secretion of bone-resorptive cytokine and also has an action to promote osteogenesis. Therefore, the agent of the present invention is a prophylactic or therapeutic agent of RA, rheumatoid-like multiple arthritis, sepsis, systemic erythematosus, systemic scleroderma, Behcet's disease, nodular periarthritis, ulcerative colitis, active chronic hepatitis, glomerular nephritis, osteoarthritis, knee osteoarthritis, gout, atherosclerosis, psoriasis, atopic dermatitis, hypercalcemia following primary hyperparathyroidism, hypercalcemia following malignant tumor, osteoporosis, and diabetes mellitus. Furthermore, the agent of the present invention is a therapeutic agent of impaired glucose tolerance for preventing or retarding the onset of NIDDM.

Preferably, the agent is a prophylactic or therapeutic agent of RA, osteoarthritis, hypercalcemia following malignant tumor, senile osteoporosis and secondary osteoporosis.

The preferable examples will now be described below. The etiology of the onset of RA is unknown, but attention is now focused on genetic predisposition with multiple factors, particularly the relation with HLA-D4, and viral infection. At an early stage, inflammation of synovial membrane is only observed, but at an advanced stage, cartilage and bone are damaged so that joints are deformed and dislocated while flexibility is lost because of bone rigidity. Systemic symptoms include multiple symptoms such as anemia, slight fever, morning stiffness, systemic malaise, easy fatigues, body weight decrease, lymphatic enlargement, and hypodermic nodules. Pathophysiologically, angiogenesis is observed in synovial membrane. The agent of the present invention is effective for the prophylaxis or therapeutic treatment of such various symptoms; in other words, the agent is effective for ameliorating or preventing the exacerbation of these symptoms.

The onset of osteoarthritis is generally occult, but degenerated meniscus is sometimes damaged in some patient with knee osteoarthritis, so that the osteoarthritis of the patient apparently seems an acute onset type. However, not any distinct cause can be found in most of such cases. The age at the onset varies more or less, depending on the site, but generally, the onset positions around 50 years old and thereafter. Major symptoms include symptoms of joint inflammation, such as joint pain (pain during motion in particular), limitation of joint flexible region, contraction of periarticular muscle, deformation of joint, topical swelling and hotness. Knee osteoarthritis occurring mainly in females is at the highest frequency; and as to osteocoxarthritis drawing next concerns, 80 to 90 % of Japanese patient is afflicted with dislocation coxarthritis and the remaining is afflicted with secondary coxarthritis due to other diseases and burn and with primary coxarthritis. The agent of the present invention is effective for the prophylaxis or therapeutic treatment of various symptoms of the aforementioned diseases; in other words, the agent is effective for ameliorating the symptoms or preventing the exacerbation of the symptoms.

Hypercalcemia following malignant tumor, as well as primary hyperparathyroidism, is typical hypercalcemia, accompanied by low PTH values in blood and frequently with high PTH-related protein values (PTHrP) in blood. So as to treat the disease, anti-tumor treatment should be done if possible. Mostly, hypercalcemia is unavoidably treated symptomatically. Patients with the disease are readily fallen into dehydration, while dehydration exacerbates hypercalcemia. The agent of the present invention is effective for the prophylaxis or therapeutic treatment of various symptoms of the aforementioned diseases; in other words, the agent is effective for ameliorating the symptoms or preventing the exacerbation of the symptoms.

Osteoporosis means the state with decrease in absolute bone level but without qualitative change in bone; and osteoporosis is divided into postmenopausal osteoporosis, senile osteoporosis and secondary osteoporosis. Postmenopausal osteoporosis is caused by the decrease in bone due to the decrease in estrogen secretion in females after menopause; senile osteoporosis is caused by the decrease in bone due to aging. Secondary osteoporosis occurs secondarily to the occurrence of specific diseases such as endocrine diseases and inflammatory diseases, irrespective of the age or sex, but the osteoporosis remits when these causes are eliminated. As to the marker of the diagnosis of osteoporosis, osteoporosis is definitely diagnosed when a state of crude longitudinal trabecule due to prominent bone atrophy is established on a lumbar X-ray image (bone atrophy degree 2), or a state with lumbar bone minerals below -2.5 standard deviation of the mean of young adult values is diagnosed by double energy X ray absorptiometry (DEXA). The agent of the present invention is effective for the prophylaxis or therapeutic treatment of such various symptoms; in other words, the agent is effective for ameliorating these symptoms or preventing the exacerbation of these symptoms.

Preferably, furthermore, the indicative diseases for which the efficacy of dienogest has already been known should be excluded. Preferably, the diseases are not subjects of the present invention. The present invention is a novel therapeutic agent of diseases which have never been believed to be indicative diseases of dienogest but for which the suppression of the production and/or secretion of bone-resorptive cytokine is effective.

Furthermore, the agent is used as the therapeutic agent after the onset, but the agent is given, in a prophylactic manner, to an individual with elevated risk factors of individual diseases prior to the onset thereof. The agent may satisfactorily be given to patients with chronic rheumatoid arthritis, in a prophylactic fashion, if these patients develop symptoms such as morning stiffness, enlargement of joints in hands and fingers, swelling of symmetric joints, hypodermic nodules and the like. In case of osteoarthritis, the agent is also given, in a prophylactic fashion, to patients with arthritis symptoms such as joint pain (pain during motion, in particular), limitation of joint flexible regions, atrophy of periarticular muscle, deformation of joint, local swelling and hotness. In case of hypercalcemia following malignant tumor, the agent is given in a prophylactic fashion to malignant tumor patients with low PTH values in blood and high PTH-related protein (PTHrP) which is observed frequently. For osteoporosis, the agent is given in a prophylactic fashion to individuals with lack of exercise, low calcium intake, disorder in menstruation, low body weight, smoking, and congenital risk factors of low bone level and to aged individuals with bone densities below a certain level.

The agent of the present invention may satisfactorily be used as a reagent at tests and laboratory tests. Specifically, the agent is useful for the method for determining the presence or absence of a specific disease or whether or not a specific substance is present in body fluids, tissues or excretes, by detecting the suppression of cytokine production in a subjective system by using the reagent.

The effects of the agent of the present invention are now specifically described in the following examples.

### Experimental Example 1

### Action on murine osteoblast

### (1) Collection and culturing of osteoblast

According to the method by Johansen, et al. (Journal Bone and Mineral Research, Vol.7, pp.501-509, 1992), osteoblast was collected from the femoral bones of a female SLC : ICR mouse of age 15 weeks. Under anesthesia with pentobarbital sodium (25 mg/kg, intraperitoneal administration), postcava was cut to bleed and kill the animal to death. Then, bilateral femoral bones were resected. This bone was cut with a pair of bone scissors, to remove the bone marrow with a micro-loop. The inner face of the bone was rubbed off with a sharp blade. The resulting matter was agitated in a 10 % collagenase solution (Nitta gelatin) at 37 °C for 20 minutes, and to the resulting mixture was added a culture medium (an MEM culture medium supplemented with 10 % charcoal-treated bovine fetal serum; manufactured by Nissui Pharmaceutical, Co. Ltd.) prior to centrifugation (300×g, 5 minutes and 20 °C). The resulting cells were dispersed in a fresh culture medium and then inoculated on a plastic culture dish (diameter of 35 mm, Primaria (Falcon)). At 37 °C in 5 % CO₂/95% air, the cells were cultured for 24 hours, and the attached cells were recovered in a 0.25 % trypsin solution to prepare a cell suspension of 1×10⁴ cells/ml. Osteoblast was identified by using alkali phosphatase staining as an osteoblast marker. For recovering osteoblast from an animal after ovariectomy, ovariectomy was carried out at age 11 weeks, and the animal was fed for the following 4 weeks, prior to the same treatment.

### (2) Pharmaceutical treatment

Dienogest as a test substance was dissolved and diluted in dimethylsulfoxide (DMSO, manufactured by Wako Pure Chemical Industries, Ltd.) for use. As a control substance, furthermore, MPA and danazol were used after they were dissolved and diluted in DMSO. DMSO was used as a solvent control.

1×10⁴ cells/ml osteoblast suspension (100 µl), a culture medium (890 µl) and a drug solution (10 µl) were added into each well of a plastic 24-well culture plate (Falcon), for culturing at 37 °C in 5 % CO₂/95 % air for 24 hours, and then, the resulting culture was centrifuged (300 ×g, 5 minutes, 20°C) to recover the supernatant, which was defined as a cytokine assay sample. The final concentrations of the drug were 10⁻¹¹, 10⁻¹⁰ and 10⁻⁹ M. For experiments, furthermore, three dishes per each group were used.

### (3) Assay of cytokine in culture supernatant

Using SMRART ™ (Pharmacia Biotech, Uppsala, Sweden), the supernatant was concentrated by about 10 fold, after the termination of culturing. The level of cytokine in the sample was assayed by using assay kits of murine IL-1β, IL-6 and TNF-α (Cytoscreen™, BioSource International, Camarillo, Calif. CA, USA) and europium-labeled streptoavidin (Pharmacia Biotech). The results are shown in Tables 1 to 3.

**Table 1**

| Effects on IL-1β release from murine osteoblast | | | |
|---|---|---|---|
| | | IL-1β (pg/ml) | |
| Drugs | Dose (M) | Non-treated mouse | Ovariectomized mouse |
| Control | - | 13.9 | 26.3 |
| Dienogest | 10⁻¹¹ | 14.4 | 26.1 |
| | 10⁻¹⁰ | 13.7 | 23.8* |
| | 10⁻⁹ | 12.0** | 23.1** |
| MPA | 10⁻¹¹ | 13.3 | 26.3 |
| | 10⁻¹⁰ | 14.4 | 25.9 |
| | 10⁻⁹ | 16.9* | 30.2** |
| Danazol | 10⁻¹¹ | 13.6 | 25.2 |
| | 10⁻¹⁰ | 13.8 | 26.0 |
| | 10⁻⁹ | 15.4* | 28.1** |
| The data was analyzed by Dunnett's test. Significant differences from the control; *p < 0.05, **P <0.01. | | | |

**Table 2**

| Effects on IL-6 release from murine osteoblast | | | |
|---|---|---|---|
| | | IL-6 (pg/ml) | |
| Drugs | Dose (M) | Non-treated mouse | Ovariectomized mouse |
| Control | - | 39.9 | 109.0 |
| Dienogest | 10⁻¹¹ | 39.3 | 94.2 |
| | 10⁻¹⁰ | 35.5 | 47.7** |
| | 10⁻⁹ | 32.3** | 40.1** |
| MPA | 10⁻¹¹ | 38.7 | 111.4 |
| | 10⁻¹⁰ | 37.6 | 109.3 |
| | 10⁻⁹ | 45.1** | 122.5** |
| Danazol | 10⁻¹¹ | 38.9 | 111.3 |
| | 10⁻¹⁰ | 37.7 | 109.4 |
| | 10⁻⁹ | 42.0** | 124.0** |
| The data was analyzed by Dunnett's test. Significant differences from the control; **P <0.01. | | | |

**Table 3**

| Effects on TNF-α release from murine osteoblast | | | |
|---|---|---|---|
| | | TNF-α (pg/ml) | |
| Drugs | Dose (M) | Non-treated mouse | Ovariectomized mouse |
| Control | - | 23.5 | 45.0 |
| Dienogest | 10⁻¹¹ | 24.7 | 44.4 |
| | 10⁻¹⁰ | 24.4 | 29.8** |
| | 10⁻⁹ | 14.8** | 22.3** |
| MPA | 10⁻¹¹ | 25.5 | 44.6 |
| | 10⁻¹⁰ | 25.3 | 46.1 |
| | 10⁻⁹ | 31.4** | 47.6* |
| Danazol | 10⁻¹¹ | 24.0 | 45.3 |
| | 10⁻¹⁰ | 24.3 | 45.3 |
| | 10⁻⁹ | 26.1** | 47.5** |
| The data was analyzed by Dunnett's test. Significant differences from the control; *p < 0.05, **P <0.01. | | | |

The results shown in Tables 1 to 3 demonstrate that as to cytokine released from osteoblast of the mouse after ovariectomy, dienogest at a concentration of 10⁻¹⁰ M or more significantly reduced the level of cytokine in the supernatant of IL-1β, IL-6 and TNF-α. As to cytokine released from osteoblast of the non-treated mouse, dienogest reduced the cytokine level. At 10⁻⁹ M, alternatively, MPA and danazol as therapeutic agents of endometriosis significantly increased the cytokine level in all the samples.

### Experimental Example 2

### Effects on pit formation by murine osteoclast

### (1) Collection and culturing of osteoclast

Osteoclast was recovered from mice according to the method by Takada et al. (Bone Mineral, Vol.17, pp.347-359, 1992). After an ICR mouse of age 13 days was bled to death under anesthesia (pentobarbital sodium), bilateral femoral bones were resected. In an α-MEM culture medium (Gibco), the femoral bones were cut into pieces with a pair of bone scissors for 5 minutes, and the resulting pieces were agitated with a vortex mixer for 30 seconds. The cell suspension was centrifuged (1000 g, 2 min., 15 °C), and the resulting supernatant was discarded. The precipitate was suspended in an α-MEM culture medium, to adjust the suspension to a cell number of 1×10⁷ cells/ml.

### (2) Drug addition

A purchased ivory piece was processed into a disk of a diameter of 5.5 mm and a thickness of 200 µm, by using a precision low-speed cutter (manufactured by BUEHLER) and a cut blade. The disk was then treated under ultrasonication in distilled water, and was immersed in an α-MEM culture medium (culture medium) supplemented with 10 % bovine fetal serum (Dai-Nippon Pharmaceuticals, Co.) treated with charcoal. The resulting each disk was placed in each well of a 96-well culture plate (Falcon), to which was added 100 µl of the drug solution. The osteoclast suspension was inoculated at 1×10⁶ cells(100µl) thereon, for culturing at 37 °C in 5 % CO₂/95 % air for 3 days. All the drugs were adjusted to final concentrations of 10⁻¹¹, 10⁻¹⁰ and 10⁻⁹ M. Additionally, five samples per each concentration were experimented.

### (3) Assay of absorbency

Absorbency was determined by calculating a phargocytized area on the bone. More specifically, the ivory piece after the treatment with the drugs was wiped with gauze, rinsed with an ultrasonication machine for 30 minutes, and stained with an acid hematoxylin solution (Sigma Chemical Co., Ltd.) for 5 minutes. The piece was then rinsed in distilled water, and the phargocytized absorption area was measured with an image analyzer (Luzex, Nireco) at 100 magnification. Nine different parts were counted per each ivory piece, to calculate the average. The results are shown in Table 4.

**Table 4**

| Effects on pit formation due to murine osteoclast | | | | |
|---|---|---|---|---|
| Drugs | Dose (M) | | | |
| | 0 | 10⁻¹¹ | 10⁻¹⁰ | 10⁻⁹ |
| Control | 0.16 | - | - | - |
| Dienogest | - | 0.15 | 0.15 | 0.10** |
| MPA | - | 0.16 | 0.16 | 0.16 |
| Danazol | - | 0.16 | 0.15 | 0.16 |
| The data was analyzed by Dunnett's test. Significant differences from the control; **p < 0.01. | | | | |

The results shown in Table 4 show that dienogest reduces the pit formation due to the murine osteoclast and demonstrates a prominent action on the suppression of bone resorption. On the other hand, MPA and danazol did not affect pit formation.

### Experimental Example 3

### Effects on frequency of occurrence of apoptosis in murine osteoclast

### (1) Preparation of osteoclast

By a method according to the report by Takahashi et al. (Endocrinology, Vol.122, pp.1373-1382, 1988), osteoclast was prepared. More specifically, a female SLC:ICR mouse of age 7 weeks was bled and killed to death, under anesthesia with pentobarbital sodium (25 mg/kg, intraperitoneal administration), and from the mouse; her femoral bone was resected. Both the ends were cut, to rinse off the myeloma into α-MEM (Gibco) (1 ml). The myeloma was rinsed twice in α-MEM and suspended in an α-MEM supplemented with 10 % charcoal-treated bovine fetus serum (culture medium), to prepare a cell suspension of 1.5×10⁶ cells/ml.

### (2) Drug treatment

0.5 ml of the cell suspension was inoculated in a 24-well microtiter plate. To the wells was added 100 ng/ml parathyroid hormone (Sigma Chemical Co.) for culturing in 5 % CO₂/95 % air at 37 °C for 2 days. After removing non-attached cells, each of drug solutions (at final concentrations of 10⁻¹¹, 10⁻¹⁰ and 10⁻⁹ M) was added to the culture medium, for culturing for another 7 days. Herein, three plates were used per one concentration of each drug.

### (3) Detection of apoptosis

After rinsing the cells in phosphate buffered saline (pH 7.4, PBS), the cells were peeled off in a 0.25 % trypsin solution, and used to prepare a smear sample by means of cytospin, prior to fixing in methyl alcohol at - 20 °C for 7 minutes. After rinsing in PBS, the apoptosis cells were detected by using an ApopTagPlus Insitu Apoptosis Detection kit peroxidase (Oncor, Gaithersburg, MD, USA) and a TSA-direct kit (GreenFISH, NEN Life Science Products, Boston, MA). Simultaneously, the smear sample was stained with a 20 µg/ml propidium iodide (Sigma Chemical Co.) solution containing 50 U/ml RNase, at room temperature for 15 minutes, to stain the nucleus. One hundred cells with two or more nuclei were observed under a cofocal laser scanning type fluorescence microscope (MRC-1000, BioRad), to determine the number of apoptosis-positive cells developed: The results are shown in Table 5.

**Table 5**

| Effects on frequency of apoptosis occurrence in murine osteoclast | | | | |
|---|---|---|---|---|
| Drugs | Dose (M) | | | |
| | 0 | 10⁻¹¹ | 10⁻¹⁰ | 10⁻⁹ |
| Control | 4.0 | - | - | - |
| Dienogest | - | 3.7 | 5.0 | 16.3** |
| MPA | - | 4.0 | 3.7 | 5.0 |
| Danazol | - | 4.0 | 3.7 | 3.3 |
| The data was analyzed by Dunnett's test. Significant differences from the control; **p < 0.01. | | | | |

The results shown in Table 5 show that dienogest at 10⁻⁹ M significantly increases the frequency of apoptosis occurrence in osteoclast. Alternatively, MPA and danazol did not affect the frequency of apoptosis occurrence.

### Experimental Example 4

### Effects on cAMP content in murine osteoblast

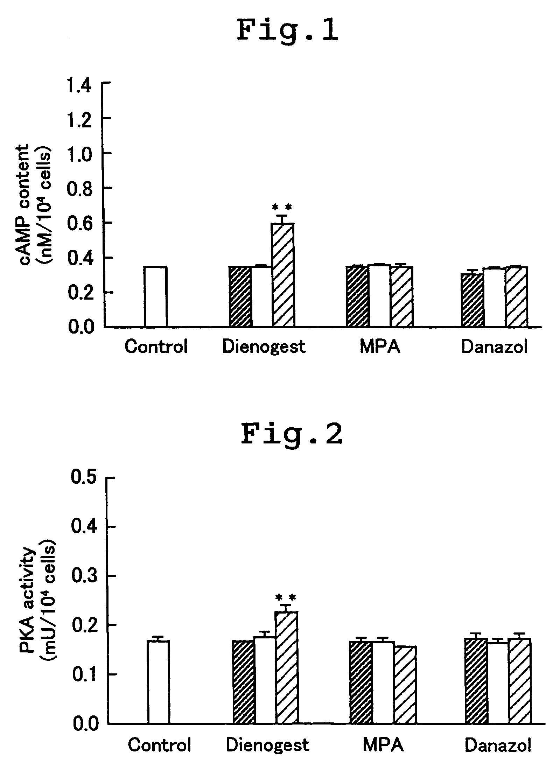

The osteoblast recovered by the method of the Experimental Example 1 was used. The conditions for drug treatment were the same as those described in the Experimental Example 1. The supernatant of the homogenate (at 10⁴ cells/ml) of the cultured cells was assayed as a sample by time-resolved fluorometry. More specifically, a goat-derived anti-rabbit IgG antibody solution (100 µl) was added to each well of a 96-well microtiter plate, for reaction at 20 °C for one hour. After the wells were rinsed with Tris buffer saline containing 0.01 % Tween 80 (rinse solution), an anti-cAMP antibody solution (100 µl) was added for reaction at 20 °C for one hour, prior to rinsing. After the reaction was blocked with a blocking solution prior to rinsing, a sample solution and a europium-labeled-cAMP-labeled protein solution (100 µl) were added into the wells. After rinsing with a DELFIA rinse solution, an enhancer reagent (100 µl) was added, for agitation at room temperature for 5 minutes, to determine the fluorescence intensity with a time-resolved fluorometer. Based on a preliminarily prepared standard curve, cAMP content was calculated. The results are shown in Fig.1. The columns of Fig.1 individually show the doses of 10⁻¹¹, 10⁻¹⁰ and 10⁻⁹ M, in this order.

The results shown in Fig.1 demonstrate that dienogest at a concentration of 10⁻⁹ M or more significantly increased cAMP content in murine osteoblast. Alternatively, MPA and danazol did not affect cAMP content at all.

### Experimental Example 5

### Effects on PKA activity in murine osteoblast

The osteoblast recovered by the method of the Experimental Example 1 was used. The conditions for drug treatment were the same as those described in the Experimental Example 1. The supernatant of the homogenate (at 10⁴ cells/ml) of the culture cells was assayed as a sample by time-resolved fluorometry. More specifically, a PKA reaction solution (MBL kit; 108 µl) was added to each well of a 96-well microtiter plate, followed by addition of a sample solution of 12 µl, for reaction at 25 °C for 5 minutes. After a reaction termination solution (100 µl) was added to the wells, the wells were rinsed five times with a rinse solution. To the wells was added a mouse-derived anti-YC antibody solution (100 µl), for reaction at 25 °C for 30 minutes, prior to rinsing. To the resulting wells was added a europium-labeled anti-mouse IgG antibody solution (100 µl), for reaction at 25 °C for 30 minutes, prior to rinsing in a DELFIA rinse solution. An enhancer reagent (100 µl) was added, for agitation at room temperature for 5 minutes, to determine the fluorescence intensity with a time-resolved fluorometer. Based on a preliminarily prepared standard curve, PKA activity was calculated. The results are shown in Fig.2. The columns of Fig.2 individually show the doses of 10⁻¹¹, 10⁻¹⁰ and 10⁻⁹ M, in this order.

The results shown in Fig.2 demonstrate that dienogest at a concentration of 10⁻⁹ M or more significantly enhanced PKA activity in the murine osteoblast, but MPA or danazol did not affect the activity at all.

As to the toxicity (safety) of the agent to suppress the production and secretion of bone-resorptive cytokine in accordance with the present invention, a possible clinical dose estimated on the basis of the present dose with in vitro efficacy is almost equal to the clinical dose of the drug already put on the use as an oral contraceptive in Germany (dose of 2 mg per day), and the safety of the drug as an agent to treat endometriosis when used singly (at a dose of 1 to 4 mg per day) has already been demonstrated at a clinical trial. Therefore, it is believed that no problem is present.

Based on the results of the experiments mentioned insofar, it is affirmed that dienogest at a concentration of 10⁻¹⁰ M or more suppresses the release of IL-1, IL-6 and TNF-α from the osteoblast of the ovariectomized mouse. For the pit formation due to murine osteoclast, dienogest exerted its suppressive effect at 10⁻⁹ M, but MPA or danazol did not exert its suppressive effect.

At 10⁻⁹ M, dienogest increased the frequency of apoptosis occurrence in osteoclast, but MPA or danazol did not modify the frequency at all, which indicates that dienogest has an action to suppress the production of bone-resorptive cytokine. Furthermore, it is indicated that dienogest at 10⁻⁹ M increases the cAMP content in osteoblast and enhances PKA activity, but MPA or danazol did not exert such actions. Thus, it is indicated that dienogest has an action to promote osteogenesis.

The dosage form of the agent of the present invention will now be described below. The agent to suppress the production and/or secretion of bone-resorptive cytokine in accordance with the present invention may be satisfactory, only if the agent contains an effective amount of at least dienogest as the effective ingredient, and the agent is generally formulated together with a pharmaceutically acceptable carrier. More specifically, the agent of the present invention is a medical or veterinary composition at least containing dienogest and a pharmaceutically acceptable carrier. The agent may satisfactorily contain appropriate additives (formulating raw materials), for example, excipients such as lactose and calcium hydrogen phosphate; binders such as powdery cellulose, dextrin and polyvinyl alcohol; disintegrators such as carboxymethyl cellulose and carboxymethyl starch; lubricants such as sucrose fatty acid ester and magnesium stearate; colorants such as pigment; flavor such as vegetable flavor; stabilizers and the like. Specific examples of the dosage form include, for example, tablets, capsules, granules, fine granules, powders, liquid solutions or suspensions, emulsions, fatty emulsions, ointments and suppositories, and additionally include sustained release formulations such as patches, tapes, and intradermal implants. The dosage form includes oral administration or parenteral administrations such as intra-rectal administration, intra-vaginal administration, transdermal absorption, trans-mucosal absorption, intravenous injection, intra-cavity articulare administration, intra-muscular administration or intra-dermal administration. Additionally, administration by way of injections or eye drops may also be included.

The dose is about 0.5 to 10 mg/day/adult, preferably 1 to 5 mg/day/adult in 1 to 5 dividend doses. Depending on the age, body weight and health status of a patient and the dosing route, the dose and dosing number may be adjusted.

The agent of the present invention may contain other effective ingredients other than dienogest as the effective ingredients thereof. Such other effective ingredients preferably include therapeutic agents of osteoporosis, anti-rheumatoid agents, and anti-tumor agents for use in treating hypercalcemia following malignant tumor. More specifically, therapeutic agents of osteoporosis include active-type vitamin D₃, calcitonin, estrogen, calcium formulations, ipriflavon, estriol, parathyroid hormone formulations, vitamin K, sodium fluoride, bisphosphonate and the like. Anti-rheumatoid agents include non-steroidal anti-inflammation drugs such as indometacin and ketoprofen; oral gold formulations such as auranofin; and penicillamine. Anti-tumor agents include alkalizing agents such as cyclophosphamide, metabolic antagonists such as amethopterin, antibiotics such as mitomycin, vegetable alkaloids and the like.

The agent of the present invention may satisfactorily be used in combination with drugs with an action to suppress the production and/or secretion of any of IL-1, IL-6 and TNF-α.

The dosage form may satisfactorily be a formulation containing dienogest and other compounds; otherwise, these may satisfactorily be administered separately. Furthermore, dienogest and the compounds may be administered concurrently to a patient; additionally, the agent may be used during the term in which conventional therapeutic agents must be reduced or terminated or must be discontinued for a while.

Examples of the present invention will be described below. The present invention is absolutely not limited to these examples.

### (Example 1)

| | |
|---|---|
| Dienogest | 2.0 g |
| Lactose | 87.0 g |
| Corn starch | 6.0 g |
| Magnesium stearate | 5.0 g |

The ingredients are mixed together, and the resulting mixture is sealed in capsule No.3 according to Japanese Pharmacopoeia, to prepare capsules each containing 100mg of the mixture.

### (Example 2)

| | |
|---|---|
| Dienogest | 0.4 g |
| Lactose | 91.6 g |
| Corn starch | 50.0 g |
| Talc | 3.0 g |
| Magnesium stearate | 5.0 g |

The ingredients are appropriately mixed together, to prepare tablets by wet granule compress process. Each tablet (150 mg) contains 0.40 mg of dienogest.

### (Example 3)

| | |
|---|---|
| Dienogest | 1.0 g |
| Polysorbate 80 | 1.0 g |
| Witepzol (S-55) | 98.0 g |

The ingredients are kneaded together under heating, and the resulting mixture is sealed in a plastic package, to prepare suppositories each of a weight of 1.0 g.

### Industrial Applicability

The agent to suppress the production and/or secretion of bone-resorptive cytokine of the present invention has a strong activity to suppress the production and/or secretion of bone-resorptive cytokine, and can exert prophylactic or therapeutic effects on diseases such as RA, osteoarthritis, hypercalcemia following malignant tumor and osteoporosis, which are caused due to the abnormality in the production and/or secretion of IL-1, IL-6 and TNF-α. The agent of the present invention can elevate RA factor CRP and improve laboratory test results of fibrinogen, platelet number anemia and reduced albumin, and can also ameliorate subjective symptoms such as pain and swelling. Furthermore, the agent can suppress the activity of osteoclast, so the agent may serve as an RA therapeutic agent. Furthermore, the agent of the present invention has not only an action to suppress bone resorption but also an action to promote osteogenesis, and therefore, the agent may function as a therapeutic agent of senile osteoporosis and secondary osteoporosis. Furthermore, the agent of the present invention can ameliorate bone reduction as a side effect of danazol. The agent of the present invention can be used singly, and additionally, the agent can be used in combination with conventional therapeutic agents of osteoporosis, anti-rheumatoid arthritis, and therapeutic agents of hypercalcemia. The agent of the present invention is additionally useful as a reagent. Still furthermore, the agent of the present invention can be administered, with less side effects, for a long time.

## Claims

1. An agent for suppressing the production and/or secretion of IL-1, IL-6 or TNF-α, wherein the agent contains dienogest or a solvate thereof as the effective ingredient.

2. An agent for suppressing the production and/or secretion of bone-resorptive cytokine, wherein the agent contains dienogest or a solvate thereof as the effective ingredient.

3. A prophylactic and/or therapeutic agent of diseases derived from the disorder in production and/or in secretion of IL-1, IL-6 or TNF-α, wherein the agent contains dienogest or a solvate thereof as the effective ingredient.

4. A prophylactic and/or therapeutic agent of hypercalcemia following chronic rheumatoid arthritis, osteoarthritis or malignant tumor, wherein the agent contains dienogest or a solvate thereof as the effective ingredient.

5. A prophylactic and/or therapeutic agent of senile osteoporosis or secondary osteoporosis, wherein the agent contains dienogest or a solvate thereof as the effective ingredient.

6. An agent for reducing side effects, wherein the agent is used in combination with danazol and the agent contains dienogest or a solvate thereof as the effective ingredient.

7. A prophylactic and/or therapeutic method of diseases due to abnormal production and/or secretion of IL-1, IL-6 or TNF-α, using a pharmaceutical composition which contains dienogest or a solvate thereof as the effective ingredient.

8. A method for reducing side effects of danazol using a pharmaceutical composition which contains dienogest or a solvate thereof as the effective ingredient.

9. A use of dienogest or a solvate thereof for the production of a prophylactic and/or therapeutic agent of diseases due to abnormal production and/or secretion of IL-1, IL-6 or TNF-α.

10. A use of dienogest or a solvate thereof for the production of an agent for reducing side effects of danazol.
